(19) Europäisches Patentamt
European Patent Office
Office européen des brevets

(11) Publication number: **0 039 920**
**B1**

(12) # EUROPEAN PATENT SPECIFICATION

(45) Date of publication of patent specification: **25.07.84**

(21) Application number: **81103498.2**

(22) Date of filing: **07.05.81**

(51) Int. Cl.³: **C 07 D 487/04,**
C 07 D 471/14,
A 61 K 31/495
//C07D471/04, (C07D487/04, 249/00, 241/00), (C07D471/14, 249/00, 241/00, 221/00), (C07D471/04, 249/00, 241/00)

(54) **Triazaloquinoxalin-1,4-diones.**

(30) Priority: **09.05.80 US 148314**

(43) Date of publication of application:
**18.11.81 Bulletin 81/46**

(45) Publication of the grant of the patent:
**25.07.84 Bulletin 84/30**

(84) Designated Contracting States:
**BE CH DE FR GB IT LI LU NL SE**

(56) References cited:
**DD - A - 126 390**

**DIE PHARMAZIE, vol. 32, no. 11, November 1977 Berlin, DE. G. WESTPHAL: "Potentielle Virostatica", pages 687-689**

**CHEMICAL ABSTRACTS, vol. 81, no. 23, 09-12-1974, page 535, abstract 152214h Columbus, Ohio, U.S.A.**

**CHEMICAL ABSTRACTS, vol. 53, no. 22, 25-11-1959, column 21979 f-i Columbus, Ohio, U.S.A.**

(73) Proprietor: **USV PHARMACEUTICAL CORPORATION**
**1 Scarsdale Road**
**Tuckahoe New York (US)**

(72) Inventor: **Brown, Richard E.**
**16 Ridge Drive**
**East Hanover New Jersey (US)**
Inventor: **Georgiev, Vasil St.**
**60 Locust Avenue**
**New Rochelle New York (US)**
Inventor: **Kropp, Philip**
**104 Kent Court**
**Meriden, Connecticut (US)**
Inventor: **Loev, Bernard**
**42 Penny Lane**
**Scarsdale, New York (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 039 920 B1

## Description

This invention relates to new anti-allergic agents and more particularly to certain new triazoloquinoxalin-1,4-diones possessing useful anti-allergic activity of particular use in the treatment of asthma.

The traizoloquinoxalin-1,4-diones of this invention are new compounds not previously described in the literature and show significant anti-allergy activity as shown in standard tests used for evaluation of such activity. These compounds show particularly significant activity in inhibiting formation of a wheal when screened according to the Rat Passive Cutaneous Anaphylaxis Screen [I. Mota, Life Science, *7*, 465 (1963) and Z. Ovary, et al., Proceedings of Society of Experimental Biology and Medicine, *81*, 584 (1952)]. The present new compounds also demonstrate potent activity as inhibitors of histamine release from passively sensitized Rat Mast cells according to the procedure described by E. Kusner, et al., Journal of Pharmacology and Therapeutics. Thus, the present new compounds are especially useful in the treatment of asthma and other allergic reactions.

The new compounds of this invention are of the following formulae:

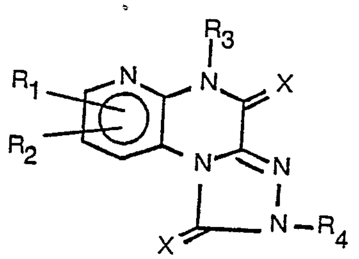

I

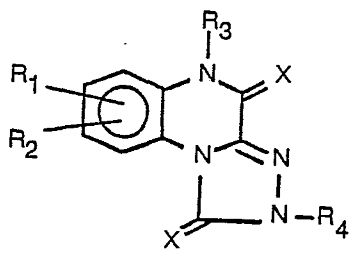

II

wherein,

X is S or O; each of $R_1$ and $R_2$ is hydrogen, $C_1$—$C_{10}$ alkyl, $C_2$—$C_{10}$ alkenyl, $C_2$—$C_{10}$ alkynyl, cyclo $C_3$—$C_{10}$ alkyl, $C_6$—$C_{10}$ aryl, $C_6$—$C_{10}$ ar $C_1$—$C_{10}$ alkyl, sulfonamido, halogen, $C_1$—$C_{10}$ alkoxy, $C_2$—$C_{10}$ alkenyloxy, $C_2$—$C_{10}$ alkynyloxy, cyano, hydroxy, nitro, amino, $C_1$—$C_{10}$ alkylamino, $C_1$—$C_{10}$ alkanoylamino, carb $C_1$—$C_{10}$ alkoxyamino, methansulfonyl, carboxy, carb $C_1$—$C_{10}$ alkoxy, trihalomethyl, or taken together, methylenedioxy;

each of $R_3$ and $R_4$ is hydrogen, $C_1$—$C_{10}$ alkyl, $C_6$—$C_{10}$ aryl, $C_{2-5}$-alkanoyl, benzoyl, $C_6$—$C_{10}$ ar $C_1$—$C_{10}$ alkyl, cyclo $C_3$—$C_{10}$ alkyl, cyclo $C_3$—$C_{10}$ alkyl $CH_2$—, or carb $C_1$—$C_{10}$ alkoxy;
and the acid addition salts thereof.

The preferred compounds are of the formula

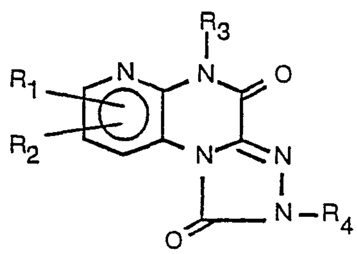

wherein

each of $R_1$ and $R_2$ is hydrogen, $C_1$—$C_{10}$ alkyl, $C_2$—$C_{10}$ alkenyl, $C_2$—$C_{10}$ alkynyl, cyclo $C_3$—$C_{10}$ alkyl, $C_6$—$C_{10}$ aryl, $C_6$—$C_{10}$ ar $C_1$—$C_{10}$ alkyl, sulfonamido, halogen, $C_1$—$C_{10}$ alkoxy, $C_2$—$C_{10}$ alkenyloxy, $C_2$—$C_{10}$ alkynyloxy, cyano, hydroxy, nitro, amino, $C_1$—$C_{10}$ alkylamino, $C_1$—$C_{10}$ alkanoylamino, carb $C_1$—$C_{10}$ alkoxyamino, methanesulfonyl, carboxy, carb $C_1$—$C_{10}$ alkoxy, trihalomethyl, or taken together, methylenedioxy; and

each of $R_3$ and $R_4$ is hydrogen, $C_1$—$C_{10}$ alkyl, $C_6$—$C_{10}$ aryl, $C_{2-5}$ alkanoyl, benzoyl, $C_6$—$C_{10}$ ar $C_1$—$C_{10}$ alkyl, cyclo $C_3$—$C_{10}$ alkyl, cyclo $C_3$—$C_{10}$ alkyl $CH_2$—, or carb $C_1$—$C_{10}$ alkoxy;
and acid addition salts thereof.

The particularly preferred compounds of the invention are those in which X is oxygen, that is the triazoloquinoxalin-1,4-diones.

The new compounds of the invention can be prepared by art-recognized procedures from known starting compounds. For example, the following procedure can be employed for compounds of formula I.

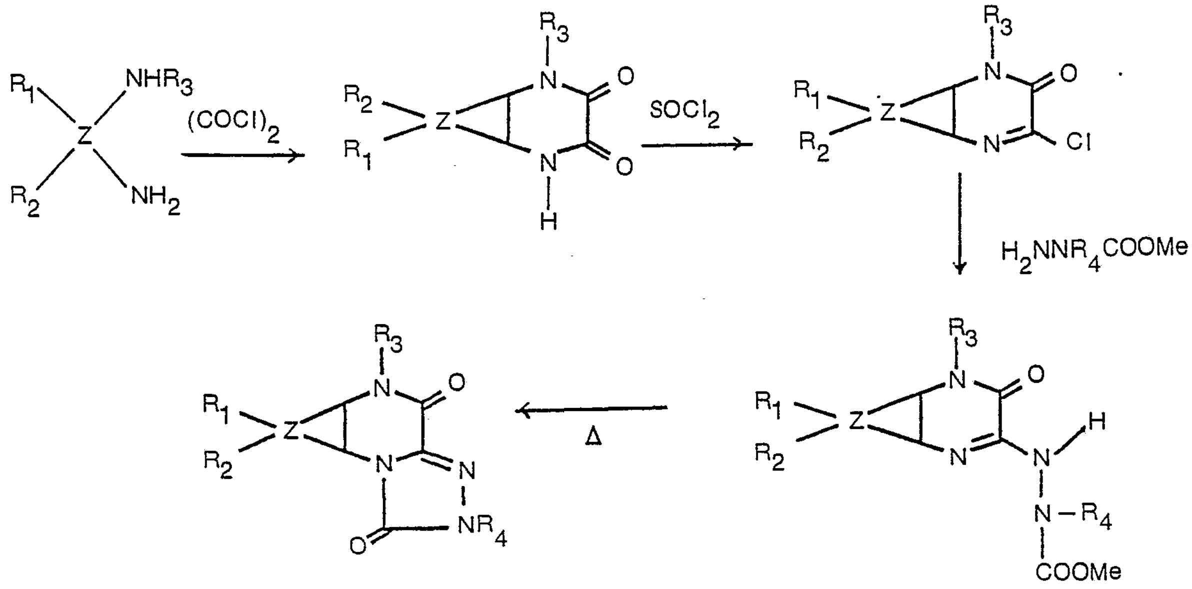


The sulfur analogs and compounds of Formula II can be prepared by analogous procedures.

Substituents $R_1$ to $R_4$ can be added after formation of the basic ring structures by known substitution reactions, or by conversion of substituents such as reduction of nitro to form amino. The substitution reactions mentioned include, for example, alkylation and acylation by known procedures.

Substituents on the present new compounds which are reactive and could interfere with ring closure reactions are best introduced by subsequent reactions known to the art such as reduction of nitro to amino, or hydrolysis of cyano to carboxamide or carboxy groups; alternatively, such reactive groups can be protected as by, for example, acylation of an amino group, followed by hydrolysis after ring closure.

Using the procedures described, a wide variety of heterocyclic compounds can be prepared as follows:

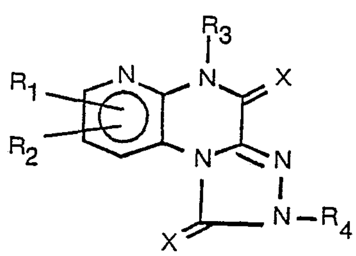

I

| $R_1$ | $R_2$ | X | $R_3$ | $R_4$ |
|---|---|---|---|---|
| Cl | H | S | H | $C_6H_5$ |
| $C_6H_5$ | H | O | $C_6H_{11}$ | $CH_3C_6H_4$ |
| $C_2H_4NH_2$ | H | O | H | H |
| H | H | O | $CH_3$ | H |
| H | H | O | $CH_3$ | H |
| H | H | O | $CH_3$ | H |

The present new heterocyclic compounds are therapeutically useful as such or can be employed in the form of salts in view of their basic nature. Thus, these compounds form salts with a wide variety of acids, inorganic and organic, including therapeutically-acceptable acids. The salts with therapeutically-acceptable acids are, of course, useful in the preparation of formulations where water solubility is desired. The salts with therapeutically-unacceptable acids are particularly useful in the isolation and purification of the present new compounds. Therefore, all acid salts of the present new compounds are contemplated by the present invention.

The pharmaceutically-acceptable acid addition salts are of particular value in therapy. These include salts of mineral acids such as hydrochloric, hydroiodic, hydrobromic, phosphoric, metaphosphoric, nitric and sulfuric acids, as well as salts of organic acids such as tartaric, acetic, citric, malic, benzoic, glycollic, gluconic, succinic, aryl-sulfonic, e.g., p-toluenesulfonic acids. The parmaceutically-unacceptable acid addition salts, while not useful for therapy, are valuable for isolation and purification of the new substances. Further, they are useful for the preparation of pharmaceutically-acceptable salts. Of this group, the more common salts include those formed with hydrofluoric and perchloric acids. Hydrofluoride salts are particularly useful for the preparation of the pharmaceutically-acceptable salts, e.g., the hydrochlorides, by solution in hydrochloric acid and crystallization of the hydrochloride salt formed. The perchloric acid salts are useful for purification and crystallization of the new products.

As therapeutic agents, the present new heterocyclic compounds are particularly useful as anti-allergy agents, acting via inhibition of mediator release. These compounds are active orally in the passive cutaneous anaphylaxis (PCA) screen; and/or inhibit histamine release from passively sensitized rat mast cells.

The therapeutic agents of this invention may be administered alone or in combination with pharmaceutically-acceptable carriers, the proportion of which is determined by the solubility and chemical nature of the compound, chosen route of administration and standard pharmaceutical practice. For example, they may be adminstered orally or in the form of tablets or capsules containing such excipients as starch, milk sugar, certain types of clay and so forth. They may be administered orally in the form of solutions which may contain coloring and flavoring agents or they may be injected parentally, that is, intramuscularly, intravenously or subcutaneously. For parenteral administration, they may be used in the form of a sterile solution containing other solutes, for example, enough saline or glucose to make the solution isotonic.

The physician will determine the dosage of the present therapeutic agents which will be most suitable and it will vary with the form of administration and the particular compound chosen, and furthermose, it will vary with the particular patient under treatment. He will generally wish to initiate treatment with small dosages substantially less than the optimum dose of the compound and increase the dosage by small increments until the optimum effect under the circumstances is reached. It will generally be found that when the composition is administered orally, larger quantities of the active agent will be required to produce the same effect as a smaller quantity given parenterally. The compounds are useful in the same manner as other anti-allergy agents and the dosage level is of the same order of magnitude as is generally employed with these other therapeutic agents. The therapeutic dosage will generally be from 10 to 750 milligrams per day and higher although it may be administered in several different dosage units. Tablets containing from 10 to 250 mg. of active agent are particularly useful.

The intermediate carbazate compounds which, on ring closure, form the new therapeutic agents of this invention are compounds which are represented by the formulae:

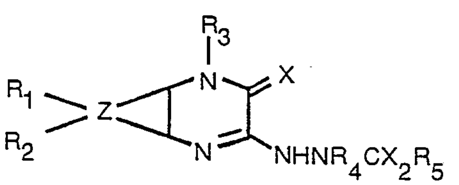

and

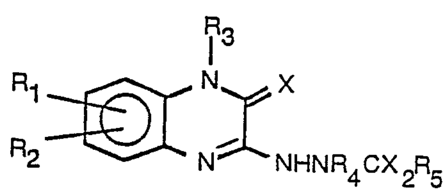

wherein $R_1$, $R_2$, $R_3$, $R_4$ and X and are as hereinbefore described; $R_5$ is alkyl, preferably lower alkyl and Z forms a pyridine ring with the two carbon atoms to which it is attached.

The new carbazate intermediates are prepared by art-recognized procedures as described herein. The intermediates also possess anti-allergic activity.

The following examples further illustrate the invention.

Example 1

5-Methyl-pyrido-(2,3-e)(1,2,4)triazolo(4,3-a)pyrazine-1,4-(2H,5H)dione

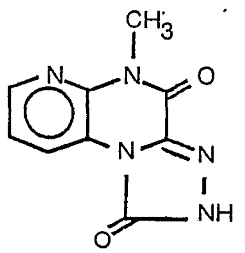

A mixture of 7.5 g of 2-chloro-4-methyl-pyrido(2,3-b)pyrazine-3-one, and 4 g. of methyl carbazate in 200 ml of Dowtherm A was stirred and heated at 80°C. for 1 hour and then at 230°C for 20 minutes.

It was then cooled and filtered. The crude product was crystallized twice from acetic acid — $H_2O$ to give the title compound in pure form, m.p.>300°C.

Example 2

2-Acetyl-5-methyl-pyrido(2,3-e)(1,2,4)triazolo(4,3-a)pyrazine-1,4-(2H,5H)dione

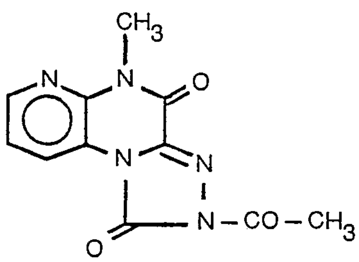

A mixture of 1.8 g of 5-methyl-pyridio(2,3-e)(1,2,4)-triazolo(4,3-a)pyrazine-1,4-(2H,5H)dione in 25 ml. of acetic anhydride and 50 ml. of acetic acid was stirred and refluxed for 5 hours.

The mixture was cooled, filtered, and the product was washed with water, dried and crystallized from acetic acid-ether, m.p. 298—300°C.

Example 3

A. 1-Methylquinoxaline-2,3-dione

To a solution of 31 g. (0.36 m.) of oxylachloride in 50 ml. *o*-dichlorobenzene at 60° is added dropwise with stirring a solution of 31.3 g (0.256 m.) N-methyl-*o*-phenylene-diamine in 150 ml. of *o*-dichlorobenzene. The mixture is then heated over 1 hour to 160°C. and cooled to 20°C. The precipitated product is filtered, washed and dried, yield 82%, m.p. 277—286°C. It may be purified by recrystallization from methanol, m.p. 286—289°C.

B. 3-Chloro-1-methyl-1H-quinoxalin-2-one

A mixture of 56.1 g. (0.32 m.) of 1,4-dihydro-1-methylquinoxalin-2,3-dione, 10 ml. of DMF, 50 ml. (0.67 m.) of thionyl chloride and 1 l. of toluene was refluxed for 1 hour, cooled, and stripped, finally under high vacuum to a crude dark solid, m.p. 124—130°C. of sufficient purity to use in the next step.

C. Ethyl-3-(1-methyl-2-oxo(2H)quinoxalin-3-yl)carbazate

A solution of 152 g. (0.78 m.) of 3-chloro-1-methyl-1H-quinoxalin-2-one, 116 g. (1.1 m.) of ethyl carbazate and 1 l. of acetonitrile is refluxed for 16 hours. The mixture is cooled to 9°C. and the product collected by filtration and used directly for cyclization.

In the same manner, the following carbazate compound are obtained:

Ethyl-3-(2-oxo(2H)quinoxalin-3-yl)carbazate
Ethyl-3-(1-phenyl-2-oxo(2H)quinoxalin-3-yl)carbazate
Ethyl-3-(1,6-dimethyl-2-ono(2H)quinoxalin-3-yl)carbazate
Ethyl-3-(1,7-dimethyl-2-oxo(2H)quinoxalin-3-yl)carbazate
Ethyl-3-(1,8-dimethyl-2-oxo(2H)quinoxalin-3-yl)carbazate
Ethyl-3-(1-methyl-6-methoxy-2-oxo(2H)quinoxalin-3-yl)carbazate
Ethyl-3-(1-methyl-6-trifluoromethyl-2-oxo(2H)quinoxalin-3-yl)carbazate

Ethyl-3-(1-methyl-6,7-dimethoxy-2-oxo(2H)quinoxalin-3-yl)carbazate
Ethyl-3-(1-methyl-8-chloro-2-oxo(2H)quinoxalin-3-yl)carbazate
Ethyl-3-(1-methyl-6-carbomethyl-2-oxo(2H)quinoxalin-3-yl)carbazate

D. 1-Oxo-5-methyl-1H,4H(1,2,4)triazolo(4,3-a)quinoxalin-4-one

100 g. of the product of Paragraph C is heated under nitrogen with constant stirring to 200—260°C, for 30 minutes. After cooling, the yellow solid is finely powdered and extracted with 2 l. of hot acetonitrile. The insoluble material, m.p. 300°C., is analytically pure, but can be recrystallized from dimethylsulfoxide.

In the same manner, the following products are obtained:

1-Oxo-1H,4H(1,2,4)triazolo(4,3-a)quinoxalin-4-one
1-Oxo-5-phenyl-1H,4H(1,2,4)triazolo(4,3-a)quinoxalin-4-one
1-Oxo-5,8-dimethyl-1H,4H(1,2,4)triazolo(4,3-a)quinoxalin-4-one
1-Oxo-5,7-dimethyl-1H,4H(1,2,4)triazolo(4,3-a)quinoxalin-4-one
1-Oxo-5,6-dimethyl-1H,4H(1,2,4)triazolo(4,3-a)quinoxalin-4-one
1-Oxo-5,7,8-trimethyl-1H,4H(1,2,4)triazolo(4,3-a)quinoxalin-4-one
1-Oxo-5-methyl-8-methoxy-1H,4H(1,2,4)triazolo(4,3-a)quinoxalin-4-one
1-Oxo-5-methyl-8-trifluoromethyl-1H,4H(1,2,4)triazolo(4,3-a)quinoxalin-4-one
1-Oxo-5-methyl-7,8-dimethoxy-1H,4H(1,2,4)triazolo(4,3-a)quinoline-4-one
1-Oxo-5-methyl-6-chloro-1H,4H(1,2,4)triazolo(4,3-a)quinoxalin-4-one
1-Oxo-5-methyl-8-carbomethoxy-1H,4H(1,2,4)triazolo(4,3-a)quinoxaline-4-one
1-Oxo-5-methyl-8-carboxy-1H,4H(1,2,4)triazolo(4,3-a)quinoxalin-4-one

## Example 4

1-Oxo-5-methyl-1H,4H(1,2,4)triazolo(4,3-a)quinoxalin-4-one

15 g. of the product of Paragraph C, Example 3 was added with stirring to Dowtherm A (200 ml.) at 230°C. The reaction mixture was kept at this temperature until cyclization was completed (usually less than 20 minutes at this or higher temperature).

Upon completion of cyclization, the reaction mixture was cooled, filtered, and the solid washed well with ethanol and methylene dichloride.

## Example 5

1-Oxo-2-acetyl-5-methyl-1H,4H(1,2,4)triazolo(4,3-a)quinoxalin-4-one

A mixture of 2.16 g. (.01 mol) of the product of Paragraph D, Example 3, 30 ml. of pyridine and 30 ml. of acetic anhydride was refluxed for 1 hour, the solvent was evaporated and the residue recrystallized from acetonitrile, m.p. 288—289°C.

## Example 6

Potassium-1-oxo-5-methyl-1H,4H(1,2,4)triazolo(4,3-a)quinoxalin-4-one-8-carboxylate

A mixture of 2.0 g of 1-oxo-5-methyl-8-carbomethoxy-1H,4H(1,2,4)triazolo(4,3-a)quinoxalin-4-one, 250 ml. of ethanol and 1 g. of potassium hydroxide was refluxed for 2 hours. The acid dissolved to give a clear solution, then a precipitate formed which was filtered, washed with alcohol and dried, m.p. 300°C.

Other compounds of formula II prepared according to the processes described above include

| $R_1$ | $R_2$ | $R_3$ | m.p. °C. |
|---|---|---|---|
| 7-chloro | H | *n*-Pr | 273—276° |
| H | H | *n*-Pr | 235—236° |
| 7-chloro | H | Me | $>300°$ |

The compounds of this invention are useful anti-allergic agents. Exemplary of the present new compounds is 6-methylpyrido(2,3-e)(1,2,4)triazolo(4,3-a)pyrazine-1,4-(2H,5H)dione which reduced wheal formation by 53% at 25 mg./kg. (p.o.) when screened according to the Rat Passive Cutaneous Anaphylaxis Screen as described by I. Mota, Life Sciences, *7*, 465 (1963) and Z. Ovary, et al., Proceedings of Society of Experimental Biology and Medicine, *81*, 584 (1952). In addition, the said compound showed an $I_{50}$ of 9.0 $\mu$M in inhibition of histamine release from passively sensitized rat mast cells according to the procedure described by E. Kusner, et al., Journal of Pharmacology by Experimental Therapeutics.

## Claims

1. An anti-allergic compound of the formulae:

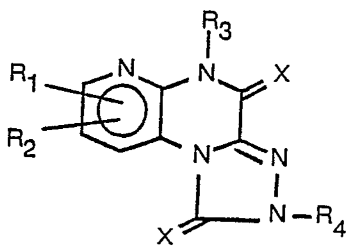

I

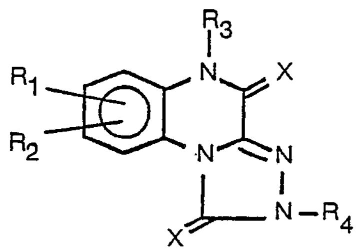

II

wherein

X is S or O; each of $R_1$ and $R_2$ is hydrogen, $C_1$—$C_{10}$ alkyl, $C_2$—$C_{10}$ alkenyl, $C_2$—$C_{10}$ alkynyl, cyclo $C_3$—$C_{10}$ alkyl, $C_6$—$C_{10}$ aryl, $C_6$—$C_{10}$ ar $C_1$—$C_{10}$ alkyl, sulfonamido, halogen, $C_1$—$C_{10}$ alkoxy, $C_2$—$C_{10}$ alkenyloxy, $C_2$—$C_{10}$ alkynyloxy, cyano, hydroxy, nitro, amino, $C_1$—$C_{10}$ alkylamino, $C_1$—$C_{10}$ alkanoylamino, carb $C_1$—$C_{10}$ alkoxyamino, methanesulfonyl, carboxy, carb $C_1$—$C_{10}$ alkoxy, trihalomethyl, or taken together, methylenedioxy;

each of $R_3$ and $R_4$ is hydrogen, $C_1$—$C_{10}$ alkyl, $C_6$—$C_{10}$ aryl, $C_{2-5}$-alkanoyl, benzoyl, $C_6$—$C_{10}$ ar $C_1$—$C_{10}$ alkyl, cyclo $C_3$—$C_{10}$ alkyl, cyclo $C_3$—$C_{10}$ alkyl $CH_2$—, or carb $C_1$—$C_{10}$ alkoxy; and the acid addition salts thereof.

2. An anti-allergic compound according to claim 1 of the formula:

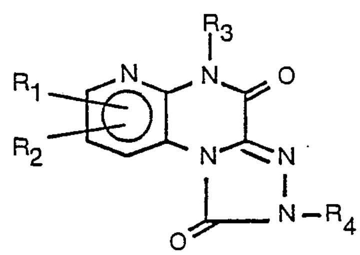

wherein

each of $R_1$ and $R_2$ is hydrogen, $C_1$—$C_{10}$ alkyl, $C_2$—$C_{10}$ alkenyl, $C_2$—$C_{10}$ alkynyl, cyclo $C_3$—$C_{10}$ alkyl, $C_6$—$C_{10}$ aryl, $C_6$—$C_{10}$ ar $C_1$—$C_{10}$ alkyl, sulfonamido, halogen, $C_1$—$C_{10}$ alkoxy, $C_2$—$C_{10}$ alkenyloxy, $C_2$—$C_{10}$ alkynyloxy, cyano, hydroxy, nitro, amino, $C_1$—$C_{10}$ alkylamino, $C_1$—$C_{10}$ alkanoylamino, carb $C_1$—$C_{10}$ alkoxyamino, methanesulfonyl, carboxy, carb $C_1$—$C_{10}$ alkoxy, trihalomethyl, or taken together, methylenedioxy; and

each of $R_3$ and $R_4$ is hydrogen, $C_1$—$C_{10}$ alkyl, $C_6$—$C_{10}$ aryl, $C_{2-5}$ alkanoyl, benzoyl, $C_6$—$C_{10}$ ar $C_1$—$C_{10}$ alkyl, cyclo $C_3$—$C_{10}$ alkyl, cyclo $C_3$—$C_{10}$ alkyl $CH_2$—, or carb $C_1$—$C_{10}$ alkoxy; and acid addition salts thereof.

3. 5-Methyl-pyrido-[2,3-e]-[1,2,4]-triazolo[4,3-a]-pyrazine-1,4-[2H,5H]-dione.

4. 2-Acetyl-5-methyl-pyrido[2,3-e]-[1,2,4]-triazolo-[4,3-a]-pyrazine-1,4-[2H,5H]-dione.

5. 5-Methyl-benzo[g]-[1,2,4]-triazolo[4,3-a]-quinoxaline-1,4-[2H,5H]-dione.

6. A therapeutical composition comprising a compound according to claim 1.

7. An anti-allergic compound according to claim 1 or the formula:

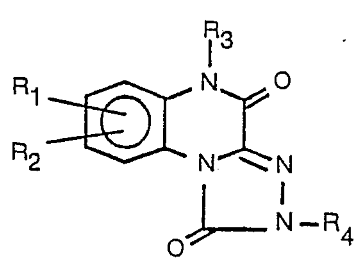

wherein,

each of $R_1$ and $R_2$ is hydrogen, $C_1$—$C_{10}$ alkyl, $C_2$—$C_{10}$ alkenyl, $C_2$—$C_{10}$ alkenyl, cyclo $C_3$—$C_{10}$ alkyl,

$C_6$—$C_{10}$ aryl, $C_6$—$C_{10}$ ar $C_1$—$C_{10}$ alkyl, sulfonamido, halogen, $C_1$—$C_{10}$ alkoxy, $C_2$—$C_{10}$ alkenyloxy, $C_2$—$C_{10}$ alkynyloxy, cyano, hydroxy, nitro, amino $C_1$—$C_{10}$ alkylamino, $C_1$—$C_{10}$ alkanoylamino, carb $C_1$—$C_{10}$ alkoxyamino, methanesulfonyl, carboxy, carb $C_1$—$C_{10}$ alkoxy, trihalomethyl, or taken together, methylenedioxy; and

each of $R_3$ and $R_4$ is hydrogen, $C_1$—$C_{10}$ alkyl, $C_6$—$C_{10}$ aryl, $C_{2-5}$ alkanoyl, benzoyl, $C_6$—$C_{10}$ ar $C_1$—$C_{10}$ alkyl, cyclo $C_3$—$C_{10}$ alkyl, cylco $C_3$—$C_{10}$ alkyl $CH_2$—, or carb $C_1$—$C_{10}$ alkoxy; and acid addition salts thereof.

8. A compound according to claim 7, wherein $R_1$ and $R_2$ are hydrogen.

9. A compound according to claim 7 wherein $R_3$ is methyl.

10. A compound according to claim 9, wherein $R_4$ is hydrogen.

11. A compound according to claim 9, wherein $R_4$ is acetyl.

12. A compound according to claim 7, wherein $R_1$ is hydrogen, $R_2$ is 8-trifluoromethyl, $R_3$ is methyl and $R_4$ is hydrogen.

13. A compound according to claim 7, wherein $R_1$ is hydrogen, $R_2$ is hydrogen, $R_3$ is methyl and $R_4$ is hydrogen.

14. A compound according to claim 7, wherein $R_1$, $R_2$, $R_3$, and $R_4$ are hydrogen.

15. A compound according to claim 7, wherein $R_1$ is hydrogen, $R_2$ is 8-chlorine, $R_3$ is methyl, and $R_4$ is hydrogen.

16. A compound according to claim 7, wherein $R_1$ is hydrogen, $R_2$ is hydrogen, $R_3$ is methyl, and $R_4$ is carbethoxy.

17. A compound according to claim 7, wherein $R_1$ is hydrogen, $R_2$ is 8-carbomethoxy, $R_3$ is methyl, and $R_4$ is hydrogen.

18. A compound according to claim 7, wherein $R_1$ is hydrogen, $R_2$ is 6-methyl, $R_3$ is methyl, and $R_4$ is hydrogen.

19. A compound according to claim 7, wherein $R_1$ is 7-chlorine, $R_2$ is hydrogen, $R_3$ is n-propyl, and $R_4$ is hydrogen.

20. A compound according to claim 7, wherein $R_1$, $R_2$ and $R_4$ are each hydrogen and $R_3$ is n-propyl.

21. A compound according to claim 7, wherein $R_1$ is 7-chloro, $R_2$ and $R_4$ are hydrogen, and $R_3$ is methyl.

**Patentansprüche**

1. Antiallergisch wirkende Verbindungen der Formeln:

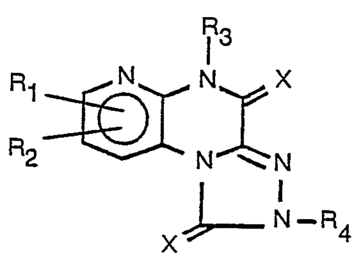

I

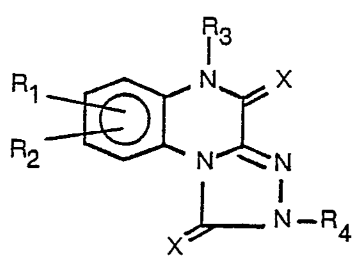

II

worin bedeuten:

X S oder O;

$R_1$ und $R_2$ jeweils Wasserstoff, $C_1$—$C_{10}$-Alkyl, $C_2$—$C_{10}$-Alkenyl, $C_2$—$C_{10}$-Alkinyl, Cyclo-$C_3$—$C_{10}$-alkyl, $C_6$—$C_{10}$-Aryl, $C_6$—$C_{10}$-Ar-$C_1$—$C_{10}$-alkyl, Sulfonamido, Halogen, $C_1$—$C_{10}$-Alkoxy, $C_2$—$C_{10}$-Alkenyloxy, $C_2$—$C_{10}$-Alkinyloxy, Cyano, Hydroxy, Nitro, Amino, $C_1$—$C_{10}$-Alkylamino, $C_1$—$C_{10}$-Alkanoylamino, Carb-$C_1$—$C_{10}$-alkoxyamino, Methansulfonyl, Carboxy, Carb-$C_1$—$C_{10}$-alkoxy, Trihalogenmethyl oder zusammengenommen Methylendioxy;

$R_3$ und $R_4$ Wasserstoff, $C_1$—$C_{10}$-Alkyl, $C_6$—$C_{10}$-Aryl, $C_{2-5}$-Alkanoyl, Benzoyl, $C_6$—$C_{10}$-Ar-$C_1$—$C_{10}$-alkyl, Cyclo-$C_3$—$C_{10}$-alkyl, Cyclo-$C_3$—$C_{10}$-alkyl-$CH_2$— oder Carb-$C_1$—$C_{10}$-alkoxy; sowie die Säureadditionssalze hiervon.

2. Antiallergisch wirkende Verbindung nach Anspruch 1 der Formel:

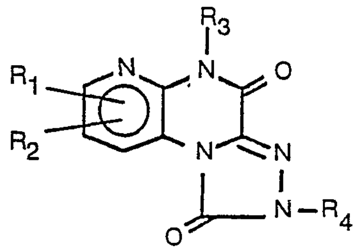

worin bedeuten:

$R_1$ und $R_2$ Wasserstoff, $C_1$—$C_{10}$-Alkyl, $C_2$—$C_{10}$-Alkenyl, $C_2$—$C_{10}$-Alkinyl, Cyclo-$C_3$—$C_{10}$-alkyl, $C_6$—$C_{10}$-Aryl, $C_6$—$C_{10}$-Ar-$C_1$—$C_{10}$-alkyl, Sulfonamido, Halogen, $C_1$—$C_{10}$-Alkoxy, $C_2$—$C_{10}$-Alkenyloxy, $C_2$—$C_{10}$-Alkinyloxy, Cyano, Hydroxy, Nitro, Amino, $C_1$—$C_{10}$-Alkylamino, $C_1$—$C_{10}$-Alkanoylamino, Carb-$C_1$—$C_{10}$-alkoxyamino, Methansulfonyl, Carboxy, Carb-$C_1$—$C_{10}$-alkoxy, Trihalogenmethyl oder zusammengenommen Methylendioxy; und

$R_3$ und $R_4$ Wasserstoff, $C_1$—$C_{10}$-Alkyl, $C_6$—$C_{10}$-Aryl, $C_{2-5}$-Alkanoyl, Benzoyl, $C_6$—$C_{10}$-Ar-$C_1$—$C_{10}$-alkyl, Cyclo-$C_3$—$C_{10}$-alkyl, Cyclo-$C_3$—$C_{10}$-alkyl-$CH_2$— oder Carb-$C_1$—$C_{10}$-Alkoxy;
sowie Säureadditionssalze hiervon.

3. 5-Methyl-pyrido-[2,3-e]-[1,2,4]-triazolo[4,3-a]-pyrazin-1,4-[2H,5H]-dion.

4. 2-Acetyl-5-methyl-pyrido[2,3,e]-[1,2,4]-triazolo-[4,3-a]-pyrazin-1,4[2H,5H]-dion.

5. 5-Methyl-benzo[g]-[1,2,4]-triazolo[4,3-a]-chinoxalin-1,4-[2H,5H]-dion.

6. Therapeutische Zusammensetzung, enthaltend eine Verbindung nach Anspruch 1.

7. Antiallergisch wirkende Verbindung nach Anspruch 1 der Formel:

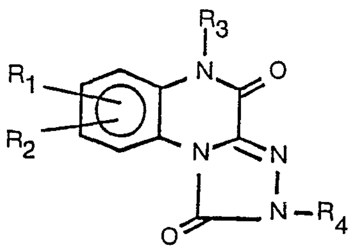

worin bedeuten:

$R_1$ und $R_2$ jeweils Wasserstoff, $C_1$—$C_{10}$-Alkyl, $C_2$—$C_{10}$-Alkenyl, $C_2$—$C_{10}$-Alkinyl, Cyclo-$C_3$—$C_{10}$-alkyl, $C_6$—$C_{10}$-Aryl, $C_6$—$C_{10}$-Ar-$C_1$—$C_{10}$-alkyl, Sulfonamido, Halogen, $C_1$—$C_{10}$-Alkoxy, $C_2$—$C_{10}$-Alkenyloxy, $C_2$—$C_{10}$-Alkinyloxy, Cyano, Hydroxy, Nitro, Amino, $C_1$—$C_{10}$-Alkylamino, $C_1$—$C_{10}$-Alkanoylamino, Carb-$C_1$—$C_{10}$-alkoxyamino, Methansulfonyl, Carboxy, Carb-$C_1$—$C_{10}$-alkoxy, Trihalogenmethyl, oder zusammengenommen Methylendioxy; und

$R_3$ und $R_4$ jeweils Wasserstoff, $C_1$—$C_{10}$-Alkyl, $C_6$—$C_{10}$-Aryl, $C_{2-5}$-Alkanoyl, Benzoyl, $C_6$—$C_{10}$-Ar-$C_1$—$C_{10}$-alkyl, Cyclo-$C_3$—$C_{10}$-alkyl, Cyclo-$C_3$—$C_{10}$-alkyl-$CH_2$—, oder Carb-$C_1$—$C_{10}$-alkoxy;
sowie Säureadditionssalze hiervon.

8. Verbindung nach Anspruch 7, dadurch gekennzeichnet, daß $R_1$ und $R_2$ Wasserstoff sind.

9. Verbindung nach Anspruch 7, dadurch gekennzeichnet, daß $R_3$ Methyl ist.

10. Verbindung nach Anspruch 9, dadurch gekennzeichnet, daß $R_4$ Wasserstoff ist.

11. Verbindung nach Anspruch 9, dadurch gekennzeichnet, daß $R_4$ Acetyl ist.

12. Verbindung nach Anspruch 7, dadurch gekennzeichnet, daß $R_1$ Wasserstof, $R_2$ 8-Trifluormethyl, $R_3$ Methyl, und $R_4$ Wasserstoff ist.

13. Verbindung nach Anspruch 7, dadurch gekennzeichnet, daß $R_1$ Wasserstoff, $R_2$ Wasserstoff, $R_3$ Methyl und $R_4$ Wasserstoff ist.

14. Verbindung nach Anspruch 7, dadurch gekennzeichnet, daß $R_1$, $R_2$, $R_3$ und $R_4$ Wasserstoffe sind.

15. Verbindung nach Anspruch 7, dadurch gekennzeichnet, daß $R_1$ Wasserstoff, $R_2$ 8-Chlor, $R_3$ Methyl und $R_4$ Wasserstoff ist.

16. Verbindung nach Anspruch 7, dadurch gekennzeichnet, daß $R_1$ Wasserstoff, $R_2$ Wasserstoff, $R_3$ Methyl und $R_4$ Carbethoxy ist.

17. Verbindung nach Anspruch 7, dadurch gekennzeichnet, daß $R_1$ Wasserstoff, $R_2$ 8-Carbomethoxy, $R_3$ Methyl und $R_4$ Wasserstoff ist.

18. Verbindung nach Anspruch 7, dadurch gekennzeichnet, daß $R_1$ Wasserstoff, $R_2$ 6-Methyl, $R_3$ Methyl und $R_4$ Wasserstoff ist.

19. Verbindung nach Anspruch 7, dadurch gekennzeichnet, daß $R_1$ 7-Chlor, $R_2$ Wasserstoff, $R_3$ n-Propyl und $R_4$ Wasserstoff ist.

20. Verbindung nach Anspruch 7, dadurch gekennzeichnet, daß $R_1$, $R_2$ und $R_4$ jeweils Wasserstoff und $R_3$ n-Propyl ist.

21. Verbindung nach Anspruch 7, dadurch gekennzeichnet, daß $R_1$ 7-Chlor, $R_2$ und $R_4$ Wasserstoff und $R_3$ Methyl ist.

## Revendications

1. Composé antiallergique répondant aux formules:

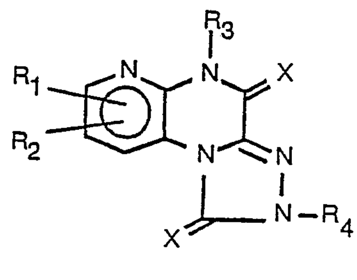

I

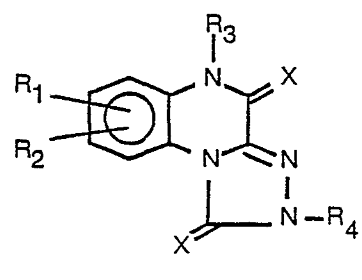

II

dans lesquelles:

X est S ou O; $R_1$ et $R_2$ sont chacun un atome d'hydrogène, un groupe alkyle en $C_1$ à $C_{10}$, alcényle en$C_2$ à $C_{10}$, alcynyle en $C_2$ à $C_{10}$, cycloalkyle en $C_3$ à $C_{10}$, aryle en $C_6$ à $C_{10}$, aryl (en $C_6$ à $C_{10}$)-alkyle (en $C_1$ à $C_{10}$) ou sulfonamido, un halogène, un groupe alcoxyle en $C_1$ à $C_{10}$, alcényloxyle en $C_2$ à $C_{10}$, alcynyloxyle en $C_2$ à $C_{10}$, cyano, hydroxyle, nitro, amine, alkylamine en $C_1$ à $C_{10}$, alcanoylamino en $C_1$ à $C_{10}$, carbalcoxylamine dont le fragment alkyle est en $C_1$ à $C_{10}$, méthanesulfonyle, carboxyle, carbalcoxyle dont le fragment alkyle est en $C_1$ à $C_{10}$, trihalogénométhyle, ou bien représentent ensemble un groupe méthylènedioxy;

$R_3$ et $R_4$ sont chacun un atome d'hydrogène, un groupe alkyle en $C_1$ à $C_{10}$, aryle en $C_6$ à $C_{10}$, alcanoyle en $C_2$ à $C_5$, benzoyle, aryl (en $C_6$ à $C_{10}$)-alkyle (en $C_1$ à $C_{10}$), cycloalkyle en $C_3$ à $C_{10}$, cycloalkyl (en $C_3$ à $C_{10}$)-$CH_2$— ou carbalcoxyle dont le fragment alkyle est en $C_1$ à $C_{10}$;

ainsi que leurs sels d'addition d'acide.

2. Composé antiallergique selon la revendication 1, répondant à la formule:

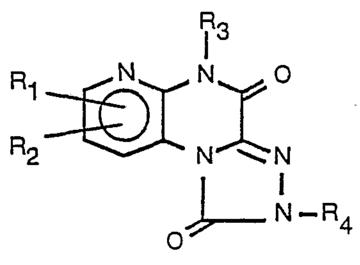

dans laquelle:

$R_1$ et $R_2$ sont chacun un atome d'hydrogène, un groupe alkyle en $C_1$ à $C_{10}$, alcényle en $C_2$ à $C_{10}$, alcynyle en $C_2$ à $C_{10}$, cycloalkyle en $C_3$ à $C_{10}$, aryle en $C_6$ à $C_{10}$, aryl (en $C_6$ à $C_{10}$)-alkyle (en $C_1$ à $C_{10}$) ou sulfonamide, un halogène, un groupe alcoxyle en $C_1$ à $C_{10}$, alcényloxyle en $C_2$ à $C_{10}$, alcynyloxyle en $C_2$ à $C_{10}$, cyano, hydroxyle, nitro, amine, alkylamine en $C_1$ à $C_{10}$, alcanoylamine en $C_1$ à $C_{10}$, carbalcoxyamine dont le fragment alkyle est en $C_1$ à $C_{10}$, méthanesulfonyle, carboxyle, carbalcoxyle dont le fragment alkyle est en $C_1$ à $C_{10}$, trihalogénométhyle, ou bien représentent ensemble un groupe méthylènedioxy; et

$R_3$ et $R_4$ sont chachun un atome d'hydrogène, un groupe alkyle en $C_1$ à $C_{10}$, aryle en $C_6$ à $C_{10}$, alkanoyle en $C_2$ à $C_5$, benzoyle, aryl (en $C_6$ à $C_{10}$)-alkyle en $C_1$ à $C_{10}$, cycloalkyle en $C_3$ à $C_{10}$, cycloalkyl (en $C_3$ à $C_{10}$)-$CH_2$— ou carbalcoxyle dont le fragment alkyle est en $C_1$ à $C_{10}$;

ainsi que leurs sels d'addition d'acide.

3. 5-méthyle-pyrido-[2,3-e]-[1,2,4]-triazolo[4,3-e]pyrazine-1,4-[2H,5H]-dione.

4. 2-acétyl-5-méthyl-pyrido[2,3-e]-[1,2,4]-triazolo-[4,3-a]-pyrazine-1,4-[2H,5H]-dione.

5. 5-méthyl-benzo[g]-[1,2,4]-triazolo[4,3-a]-quinoxaline-1,4-[2H,5H]-dione.

6. Composition thérapeutique comprenant un composé selon la revendication 1.

7. Composé antiallergique selon la revendication 1, répondant à la formule:

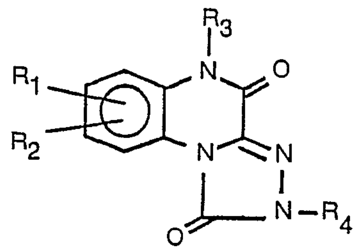

dans laquelle:

$R_1$ et $R_2$ sont chacun un atome d'hydrogène, un groupe alkyle en $C_1$ à $C_{10}$, alcényle en $C_2$ à $C_{10}$, alcynyle en $C_2$ à $C_{10}$, cycloalkyle en $C_3$ à $C_{10}$, aryle en $C_6$ à $C_{10}$, aryl (en $C_6$ à $C_{10}$)-alkyle (en $C_1$ à $C_{10}$) ou sulfonamide, un halogène, un groupe alcoxyle en $C_1$ à $C_{10}$, alcényloxyle en $C_2$ à $C_{10}$, alcynyloxyle en $C_2$ à $C_{10}$, cyano, hydroxyle, nitro, amine, alkylamine en $C_1$ à $C_{10}$, alcanoylamine en $C_1$ à $C_{10}$, carbalcoxyamine dont le fragment alkyle est en $C_1$ à $C_{10}$, méthanesulfonyle, carboxyle, carbalcoxyle dont le fragment alkyle est en $C_1$ à $C_{10}$, trihalogénométhyle, ou bien représentent ensemble un groupe méthylènedioxy; et

$R_3$ et $R_4$ sont chachun un atome d'hydrogène, un groupe alkyle en $C_1$ à $C_{10}$, aryle en $C_6$ à $C_{10}$, alcanoyle en $C_2$ à $C_5$, benzoyle, aryl (en $C_6$ à $C_{10}$)-alkyle en $C_1$ à $C_{10}$, cycloalkyle en $C_3$ à $C_{10}$, cycloalkyl (en $C_3$ à $C_{10}$)-$CH_2$— ou carbalcoxyle dont le fragment alkyle est en $C_1$ à $C_{10}$;

ainsi que leurs sels d'addition d'acide.

8. Composé selon la revendication 7, dans lequel $R_1$ et $R_2$ sont des atomes d'hydrogène.

9. Composé selon la revendication 7, dans lequel $R_3$ et un groupe méthyle.

10. Composé selon la revendication 9, dans lequel $R_4$ est un atome d'hydrogène.

11. Composé selon la revendication 9, dans lequel $R_4$ est un groupe acétyle.

12. Composé selon la revendication 7, dans lequel $R_1$ est un atome d'hydrogène, $R_2$ est un groupe 8-trifluorométhyle, $R_3$ est un groupe méthyle et $R_4$ est un atome d'hydrogène.

13. Composé selon la revendication 7, dans lequel $R_1$ est un atome d'hydrogène, $R_2$ est un atome d'hydrogène, $R_3$ est un groupe méthyle et $R_4$ est un atome d'hydrogène.

14. Composé selon la revendication 7, dans lequel $R_1$, $R_2$, $R_3$ et $R_4$ sont des atomes d'hydrogène.

15. Composé selon la revendication 7, dans lequel $R_1$ est un atome d'hydrogène, $R_2$ est un atome de chlore en 8, $R_3$ est un group méthyle et $R_4$ est un atome d'hydrogène.

16. Composé selon la revendication 7, dans lequel $R_1$ est un atome d'hydrogène, $R_2$ est un atome d'hydrogène, $R_3$ est un groupe méthyle et $R_4$ est un groupe carbéthoxyle.

17. Composé selon la revendication 7, dans lequel $R_1$ est un atome d'hydrogène, $R_2$ est un groupe 8-carbométhoxy, $R_3$ est un groupe méthyle et $R_4$ est un atome d'hydrogène.

18. Composé selon la revendication 7, dans lequel $R_1$ est un atome d'hydrogène, $R_2$ est un groupe 6-méthyle, $R_3$ est un groupe méthyle et $R_4$ est un atome d'hydrogène.

19. Composé selon la revendication 7, dans lequel $R_1$ est un atome de chlore en 7, $R_2$ est un atome d'hydrogène, $R_3$ est un groupe n-propyle et $R_4$ est un atome d'hydrogène.

20. Composé selon la revendication 7, dans lequel $R_1$, $R_2$ et $R_4$ sont chacun un atome d'hydrogène et $R_3$ est un groupe n-propyle.

21. Composé selon la revendication 7, dans lequel $R_1$ est un atome de chlore en 7, $R_2$ et $R_4$ sont des atomes d'hydrogène et $R_3$ est un groupe méthyle.